(19) Europäisches Patentamt / European Patent Office / Office européen des brevets

(11) **EP 2 185 102 B1**

(12) **FASCICULE DE BREVET EUROPEEN**

(45) Date de publication et mention de la délivrance du brevet:
**08.11.2017 Bulletin 2017/45**

(51) Int Cl.:
*A61B 5/0488* (2006.01)   *A61F 2/00* (2006.01)
*A61B 5/11* (2006.01)   *A61B 5/20* (2006.01)

(21) Numéro de dépôt: **08787000.2**

(22) Date de dépôt: **07.08.2008**

(86) Numéro de dépôt international:
**PCT/EP2008/060404**

(87) Numéro de publication internationale:
**WO 2009/027196 (05.03.2009 Gazette 2009/10)**

(54) **DISPOSITIF DE PREVENTION DE FUITES URINAIRES**

VORRICHTUNG ZUR VERHINDERUNG VON AUSTRETEN VON URIN

DEVICE FOR PREVENTING URINARY LEAKAGE

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **24.08.2007 FR 0757159**

(43) Date de publication de la demande:
**19.05.2010 Bulletin 2010/20**

(73) Titulaire: **Uromems**
**38000 Grenoble (FR)**

(72) Inventeurs:
• **CINQUIN, Philippe**
  **F-38330 St Nazaire les Eymes (FR)**
• **MOZER, Pierre**
  **F-94300 Vincennes (FR)**
• **LAMRAOUI, Hamid**
  **F-38000 Grenoble (FR)**
• **BONVILAIN, Agnès**
  **F-73800 Myans (FR)**
• **ROBAIN, Gilberte**
  **F-75005 Paris (FR)**

(74) Mandataire: **Regimbeau**
**20, rue de Chazelles**
**75847 Paris Cedex 17 (FR)**

(56) Documents cités:
**WO-A-2006/026509**

**Description**

DOMAINE DE L'INVENTION

La présente invention concerne les dispositifs de régulation artificielle de l'écoulement de fluide chez l'humain, et plus précisément les dispositifs destinés à prévenir l'incontinence urinaire.

ETAT DE LA TECHNIQUE

L'incontinence urinaire est un handicap qui touche aussi bien les femmes que les hommes. Ce handicap peut être défini comme une perte involontaire d'urine par l'urètre. Dans la plupart des cas, cela est dû à un affaiblissement du support pelvien ou du bloc vessie/sphincter.

Selon les symptômes, il existe des solutions ne nécessitant aucune intervention chirurgicale, telles que la rééducation ou des traitements médicamenteux. Lorsque ces méthodes ne suffisent pas, l'incontinence sévère nécessite alors la pose d'une prothèse pour permettre de rendre au patient une vie sociale normale. En cas d'incontinence sévère, la méthode la plus employée consiste à mettre en place un sphincter urinaire artificiel (SUA).

La prothèse la plus répandue consiste en un système hydraulique en élastomère de silicone composé de trois parties principales. La première partie consiste en une manchette occlusive placée autour de l'urètre exerçant une pression circonférentielle urétrale grâce à un coussin rempli de liquide assurant ainsi la continence du patient. La deuxième est un ballon de régulation qui permet, lorsqu'il est rempli avec un certain volume de liquide (environ 20 ml) de créer une pression hydraulique constante. La pression de régulation est choisie en fonction du patient pendant l'opération, celle-ci ne pouvant plus être modifiée une fois la prothèse en place. Il faut enfin une pompe assurant l'ouverture de la partie urétrale comprimée par la manchette. Cette pompe est composée d'une poire, d'un résistor et de deux valves qui assurent la circulation du liquide vers ou depuis la manchette occlusive. Lorsque la personne ressent le besoin d'uriner, elle comprime la poire située sur la partie inférieure de la pompe, le fluide est transféré de la manchette vers le ballon de régulation : la pression exercée sur l'urètre devient alors négligeable devant la pression vésicale. L'urine peut alors s'écouler librement hors de la vessie, ce qui en fait une méthode non dysuriante. Quelques minutes après, le liquide est transféré du ballon vers la manchette grâce à la pression exercée sur le résistor par le ballon de régulation, l'urètre est de nouveau occlus. La pompe de contrôle se trouve chez l'homme dans le scrotum et chez la femme dans l'une des grandes lèvres.

L'efficacité de cette prothèse en fait un produit de référence mais elle présente toutefois un certain nombre d'inconvénients. En premier lieu, comme on l'a déjà dit plus haut, la pression de régulation de la manchette ne peut être adaptée qu'au moment de la pose de la prothèse, ce qui pourra poser des problèmes si la pathologie évolue de sorte que la pression au niveau de l'urètre devrait être modifiée pour répondre aux besoins du patient. En outre, ce système n'offre pas un confort satisfaisant un patient qui doit actionner la pompe à chaque fois que cela est nécessaire, la commande n'étant de surcroit pas aisée puisqu'il convient de maintenir la pompe d'une part et de la presser avec force d'autre part. Enfin, le fonctionnement de cette prothèse passe par une compression importante de l'urètre en quasi continu, ce qui peut induire des atrophies urétrales. En effet, la prothèse ne fonctionnant qu'à une seule pression urétrale, celle-ci doit être assez importante pour éviter les fuites, ce qui à terme peut endommager l'urètre. Il existe enfin des problèmes de fiabilité des éléments constituant la prothèse, notamment de l'ensemble pompe et circuit hydraulique.

Des dispositifs alternatifs ont été développés pour tenter de remédier à certains de ces inconvénients, en proposant notamment des commandes électroniques de la prothèse, mais aucun de ne s'est révélé satisfaisant, notamment en ce qui concerne leur mise en oeuvre, ou la sécurité du patient. WO0150833 est considéré comme l'état de la technique le plus proche. Les inconvénients mentionnés ci-dessus liés aux sphincters urinaires artificiels existants impliquent que ces prothèses ne sont utilisées que pour traiter les incontinences urinaires importantes. En conséquence, les sphincters urinaires artificiels ne sont pas utilisés pour les patients souffrant d'une faible incontinence, c'est à dire à dire généralement uniquement à l'effort. En effet ces derniers préfèrent subir la gêne occasionnée par leur pathologie, ou opter pour une prothèse dysuriante, c'est à dire une prothèse exerçant constamment une pression sur l'urètre. Pour que la miction soit possible, le patient doit contracter suffisamment sa vessie pour lutter contre la résistance créée au niveau de l'urètre par la prothèse. Outre l'inconvénient mentionné plus haut de compression quasi-continue de l'urètre pouvant créer des atrophies urétrales, l'effort requis pour la miction avec ce type de prothèse crée une sorte d'adénome de prostate artificiel qui peut avoir des conséquences pour le patient.

Un but de la présente invention est donc de proposer un dispositif de prévention de fuites urinaires chez un patient résolvant au moins l'un des inconvénients précités.

Un but de la présente invention est plus particulièrement de proposer un dispositif de prévention de fuites urinaires dont le fonctionnement réduit la compression moyenne au niveau de l'urètre pour éviter sa détérioration.

Un autre but de l'invention est de proposer un dispositif de prévention de fuites urinaires simple et fiable, pouvant ainsi être utilisé quelque que soit la gravité de l'incontinence urinaire à traiter.

EXPOSE DE L'INVENTION

A cette fin on propose un dispositif de prévention de fuites urinaires destiné à être implanté chez un patient, comprenant :

- des moyens de compression (1) de l'urètre du patient,
- des moyens de commande électronique (2) pour actionner les moyens de compression,

caractérisé en ce qu'il comprend en outre des moyens de mesure (7) de l'activité du patient couplés aux moyens de commande (2), les moyens de commande (2) fonctionnant suivant un modèle prédictif des fuites urinaires basé sur l'activité du patient, de manière à anticiper une fuite urinaire éventuelle en fonction de l'activité mesurée du patient.

Des aspects préférés mais non limitatifs du dispositif de prévention de fuites urinaires sont les suivants :

- les moyens de mesure comprennent des moyens de mesure de la position et le déplacement du patient, lesdits moyens de mesure de la position et du déplacement du patient pouvant comprendre un accéléromètre comprenant un ou de plusieurs axes de mesure ;
- les moyens de mesure comprennent des moyens de mesure de la pression intra-abdominale du patient ;
- les moyens de mesure comprennent des moyens de mesure de la pression intra-vésicale ;
- les moyens de mesure comprennent des moyens de mesure de la pression endo-urétrale du patient ;
- les moyens de mesure comprennent des moyens de mesure de l'activité d'un muscle ;
- les moyens de mesure de l'activité d'un muscle comprennent un capteur MMG (mécanomyographe) destiné à être placé sur ledit muscle pour mesurer les mouvements généré lors de contractions dudit muscle ;
- les moyens de mesure de l'activité d'un muscle comprennent un capteur EMG (électromyographe) destiné à être placé à travers ledit muscle pour mesurer le potentiel électrique généré lors de contractions dudit muscle ;
- les moyens de mesure de l'activité d'un muscle comprennent des moyens pour la mesure de l'activité d'au moins une portion d'un des muscles grands droits, la mesure de l'activité d'au moins un des muscles pelviens, et/ou la mesure de l'activité du détrusor ;
- les moyens de mesure comprennent des moyens de mesure de la fréquence cardiaque du patient ;
- les moyens de mesure comprennent des moyens de mesure de la fréquence respiratoire du patient ;
- le dispositif caractérisé en ce qu'il comprend en outre :

    ◦ des moyens de détection destinés à être disposés chez le patient pour une détection de fuites urinaires,
    ◦ des moyens de stockage de signaux de mesure et de détection issus respectivement des moyens de mesure et des moyens de détection,
    ◦ des moyens de traitements des signaux de mesure et de détection stockés pendant un temps déterminé correspondant à une période significative de l'activité du patient, pour construire le modèle prédictif de fuites urinaires du patient, en corrélant une combinaison de tout ou d'une partie des signaux de mesure à la présence ou non d'une fuite urinaire ultérieure, de sorte que le modèle prédictif permette d'anticiper une fuite urinaire éventuelle en fonction de l'activité mesurée du patient ;

- Le dispositif comprend en outre des moyens de mesure de réplétion de la vessie du patient, le modèle prédictif des fuites urinaires étant en outre basé sur la réplétion de la vessie du patient ;
- les moyens de mesure de réplétion de la vessie comprennent un capteur échographique destiné à être implanté chez le patient pour visualiser la vessie ;
- les moyens de commande comprennent des moyens pour actionner les moyens de compression de façon dynamique en fonction de l'activité mesurée du patient ;
- les moyens de compression son adaptés pour exercer sur l'urètre une compression d'intensité variable, allant d'une absence totale de compression jusqu'à une occlusion totale de l'urètre ;
- le dispositif comprend en outre des moyens de sécurité adaptés pour actionner les moyens de compression en vue d'une absence totale de compression de l'urètre en réponse à une instruction d'ouverture, et, en réponse à une instruction de fermeture, pour actionner les moyens de compression en vue d'une compression de l'urètre égale à la compression précédant l'instruction d'ouverture du patient ;
- les moyens de sécurité sont couplés à des capteurs physiologiques destinés à être implantés chez le patient, pour permettre au patient de transmettre une instruction d'ouverture ou de fermeture aux moyens de sécurité ;
- les capteurs physiologiques sont agencés pour la mesure de contraction d'un muscle de sorte que l'instruction d'ouverture ou de fermeture soit fonction de la fréquence de contraction dudit muscle ;
- les moyens de sécurité sont couplés à des moyens de commande externes adaptés pour permettre à une tierce personne de transmettre une instruction d'ouverture ou de fermeture aux moyens de sécurité. ;
- les moyens de sécurité sont aptes à être activés par les moyens de commandes externes par ondes radio. ;

- les moyens de sécurité sont aptes à être activés par les moyens de commandes externes par ondes magnétiques ;
- les moyens de sécurité son couplés à une horloge interne, l'horloge interne permettant d'enregistrer les intervalles de temps entre les mictions du patient, les moyens de sécurité étant adaptés pour actionner les moyens de compression en vue d'une absence totale de compression de l'urètre en cas de dépassement d'un intervalle de temps maximum entre deux mictions ;

On propose également un procédé de contrôle d'un dispositif de prévention de fuites urinaires destiné à être implanté chez un patient, ledit dispositif comprenant des moyens de compression de l'urètre du patient, des moyens de commande électronique pour actionner les moyens de compression, et des moyens de mesure de l'activité du patient couplés aux moyens de commande, le procédé étant caractérisé en ce qu'il comprend les étapes consistant à :

- mesurer l'activité du patient avec les moyens de mesure,
- comparer l'activité mesurée du patient avec un modèle prédictif des fuites urinaires basé sur l'activité du patient,
- commander les moyens de compression en fonction de l'activité mesurée et du modèle prédictif pour anticiper une fuite urinaire éventuelle.

Des aspects préférés mais non limitatifs de ce procédé de contrôle sont les suivants :

- le procédé comprend en outre les étapes préalables consistant à :

  ◦ réaliser, pendant un temps déterminé correspondant à une période significative de l'activité du patient, une mesure de l'activité du patient avec les moyens de mesure, et enregistrer des signaux de mesure correspondants,
  ◦ réaliser simultanément une détection de fuites urinaires avec des moyens de détection disposés chez le patient pour une détection de fuites urinaires éventuelles, et enregistrer des signaux de détection correspondants,
  ◦ déterminer l'évolution des signaux de mesures précédant chaque fuite urinaire détectée,
  ◦ construire le modèle prédictif de fuites urinaires du patient, en corrélant un signal de mesure donné à la présence ou non d'une fuite urinaire ultérieure, de sorte que le modèle prédictif permette d'anticiper une fuite urinaire éventuelle en fonction de l'activité mesurée du patient ;

- le procédé comprend en outre une étape consistant à mesurer la réplétion de la vessie du patient, le modèle prédictif des fuites urinaires étant en outre déterminé en fonction de la réplétion de la vessie ;
- en réponse à une instruction d'ouverture, les moyens de compression n'exercent plus aucune compression sur l'urètre, et que, en réponse à une instruction de fermeture, les moyens de compression exercent une compression de l'urètre égale à la compression précédant l'instruction d'ouverture du patient, le patient pouvant donner une instruction d'ouverture ou de fermeture par un signal physiologique.

## DESCRIPTION DES FIGURES

D'autres caractéristiques et avantages de l'invention ressortiront encore de la description qui suit, laquelle est purement illustrative et non limitative et doit être lue en regard des dessins annexés, sur lesquels :

- la figure 1 est une représentation schématique du dispositif de prévention de fuite urinaire selon l'invention :
- la figure 2 illustre l'utilisation d'un accéléromètre chez un patient pour le dispositif de la figure 1 ;
- la figure 3 illustre la mesure de l'inclinaison du patient ayant un accéléromètre comme illustré à la figure 2 ;
- la figure 4 illustre les seuils de déclenchement de l'accéléromètre pour le dispositif de la figure 1.

## DESCRIPTION DETAILLEE DE L'INVENTION

Les prothèses connues pour pallier l'incontinence urinaire exercent en général une pression constante sur l'urètre sauf au moment de la miction où la pression est relâchée pour permettre une miction sans contrainte.

Toutefois, pour un patient sain, le sphincter a un fonctionnement dynamique, c'est à dire qu'il va exercer une pression plus ou moins importante sur l'urètre en fonction des besoins. La sollicitation du sphincter est en effet plus ou moins importante en fonction de l'activité exercée par le patient, mais aussi en fonction de la réplétion de la vessie (c'est à dire du volume d'urine dans la vessie).

Le dispositif proposé ici, qui va être décrit en détail ci-dessous, reproduit ce fonctionnement dynamique du sphincter, permettant de faire varier la pression sur l'urètre en fonction de l'activité du patient, de manière à réduire la pression moyenne exercée au niveau de l'urètre.

Le principe de fonctionnement du dispositif de prévention de fuites urinaires présenté est de suivre l'activité du patient à l'aide de différents capteurs pour être en mesure, à partir d'un modèle prédictif permettant d'anticiper la possibilité d'une fuite urinaire liée à l'augmentation de la pression intra-vésicale par rapport à la pression urétrale, d'exercer une pression au niveau de l'urètre lorsque cela est nécessaire pour éviter ces fuites.

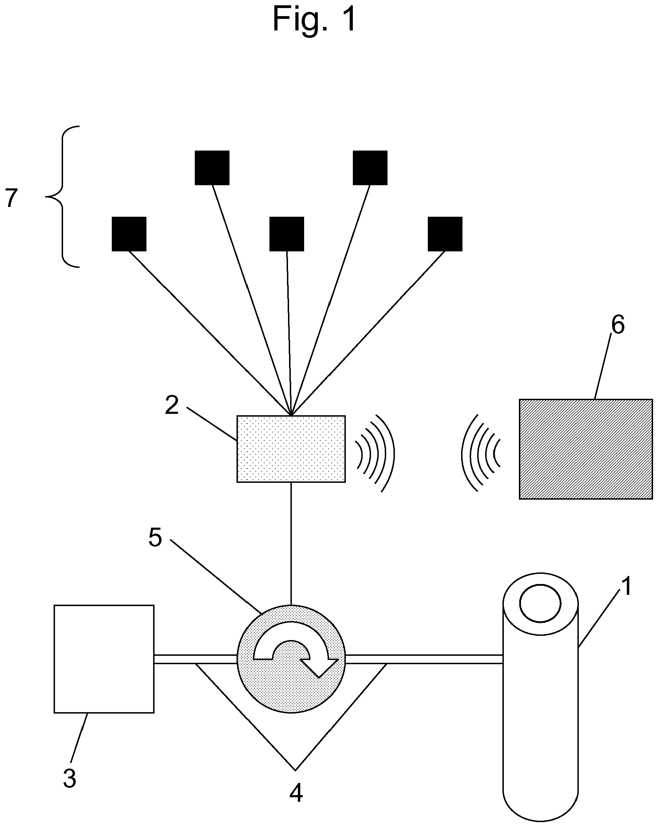

Comme on le voit illustré à la figure 1, le dispositif de prévention de fuites urinaires comprend une manchette occlusive 1 destiné à être placée autour de l'urètre du patient à traiter.

Le dispositif présenté est commandé électroniquement et comprend en conséquence un boitier de commande électronique 2, comprenant au moins un microprocesseur.

Ce boitier de commande 2 permet d'actionner la manchette 1 de sorte qu'elle exerce une pression plus au moins importante sur l'urètre. L'actionnement de la manchette 1 peut être de tout type, aussi bien hydraulique que mécanique.

Sur l'exemple illustré à la figure 1, la manchette 1 est couplée à un circuit d'actionnement hydraulique comprenant un réservoir 3 et des conduits 4 de circulation d'un fluide. Ce circuit hydraulique comprend en outre des moyens de circulation du fluide 5 permettant au fluide de circuler du réservoir 3 vers la manchette 1 et inversement.

Ce moyen de circulation 5 est adapté pour une circulation de fluide à des débits relativement élevés de plusieurs dizaines de millilitres par minute. Il convient en effet de faire circuler le fluide rapidement dans le circuit hydraulique pour que la manchette puisse être amenée à la pression désirée, en un temps relativement court, de l'ordre de 100 ms.

On pourra par exemple utiliser comme moyen de circulation 5 une micro-pompe. Pour augmenter les performances de la micro-pompe si nécessaire, il est possible de placer une chambre de surpression en sortie de la pompe avec une microvalve à commande proportionnelle à l'entrée de la manchette, ce qui permet d'atteindre la pression requise en un temps plus court. Une autre solution est d'utiliser comme moyen de circulation 5 un système à piston actionné par un micromoteur, le piston permettant d'injecter rapidement du liquide dans la manchette et d'atteindre ainsi les pressions requises.

Le dispositif de commande 2 comprend en outre des moyens pour communiquer à distance avec un dispositif de commande externe 6. Cette communication peut être effectuée par onde radio, avec par exemple une technologie de type Bluetooth©. Cela permettra de modifier le paramétrage du dispositif de commande 2, voire d'en prendre le contrôle pour forcer le fonctionnement du dispositif de prévention de fuites urinaires.

Enfin, le dispositif comprend une pluralité de capteurs 7 permettant de mesurer l'activité du patient. Ces capteurs 7 pourront être de tout type ; on pourra par exemple utiliser un accéléromètre pour mesurer les mouvements du patient, des capteurs MMG (mécanomyographe) ou EMG (électromyographes) pour mesurer l'activité de certains muscles du patient, ou encore des capteurs de pression (abdominale, urétrale, voire intra vésicale lorsque cela est possible).

Le choix des capteurs 7 sera expliqué plus en détail dans la suite de la description. Notons toutefois que les capteurs utilisés ne nécessitent pas d'intervention chirurgicale proprement dite pour leur mise en place.

Ils pourront en effet simplement être placés dans certaines parties de l'abdomen.

Ces capteurs 7 vont servir dans la phase de calibration du dispositif, au cours de laquelle un modèle prédictif des fuites urinaires va être implémenté. Ils serviront également lors de l'utilisation normale du dispositif de prévention de fuites urinaires pour permettre de faire varier la pression de la manchette 1 au niveau de l'urètre à chaque fois que cela sera nécessaire.

Le modèle prédictif des fuites urinaires est conçu pour anticiper une fuite urinaire que le patient pourrait subir, en se basant sur des informations concernant l'activité du patient. En effet, certaines activités particulières induisent une modification du rapport entre la pression intra-vésicale et la pression urétrale ce qui peut entrainer des fuites urinaires indésirées. C'est le cas par exemple lorsque le patient effectue un effort par rapport à son activité normale. C'est le cas également lorsque le patient est en phase de sommeil, où la pression urétrale diminue par rapport à la pression intra-vésicale. Une détection de ces situations particulières permet donc d'anticiper une fuite éventuelle et de modifier en conséquence la pression exercée sur l'urètre.

Pour éviter de faire varier en continu la pression exercée sur l'urètre, on s'attache à 4 phases principales au cours desquelles la pression sur l'urètre doit être modifiée.

Il y a tout d'abord la phase de miction, où le sphincter n'exerce pas ou peu de pression sur l'urètre de sorte qu'un patient peu uriner librement, sans exercer d'effort particulier. Au cours de cette phase de miction, on peut exercer une pression très faible sur l'urètre, correspondant par exemple à une pression $P_0$ dans la manchette inférieure à 10 cmH$_2$O (10 centimètres d'eau).

Comme on l'a déjà indiqué, une autre phase important correspond à la phase de sommeil, au cours de laquelle l'organisme se relâche. La pression $P_{allong}$ correspondante peut être relativement faible pouvant même avoisiner dans certains cas $P_0$. Notons que cette pression dépend du patient et pourra être fixée au cas par cas une fois le dispositif installé.

La phase la plus courante correspond au moment où le patient a une activité normale, c'est à dire au cours de laquelle il n'exerce pas d'effort particulier (posture debout, assise, en train de marcher, etc.). Dans ce cas une pression $P_{act}$ doit être exercée sur l'urètre. Cette pression doit être déterminée au cours du calibrage du dispositif de manière à être tout juste suffisante pour éviter les fuites.

La dernière phase correspond au moment où le patient exerce un effort qui implique des pressions intra-vésicales élevées (sport, toux, etc.). La pression $P_{effort}$ exercée sur l'urètre est dans ce cas plus importante que la pression $P_{act}$. $P_{effort}$ permet en fait de moduler $P_{act}$, lors d'une augmentation brutale de la pression dans la vessie du patient qui serait en activité normale ; par exemple après une toux, la pression dans la manchette passe de $P_{act}$ à $P_{effort}$ pendant un instant puis repasse à $P_{act}$ si la pression vésicale a diminuée.

Bien sûr, l'activité du patient et la pression du sphincter associée pourrait être découpée en bien d'autres phases. Le dispositif proposé est décrit pour un contrôle dynamique de la pression au niveau de l'urètre selon ces 4 phases, même s'il pourrait être aisément adapté pour un autre découpage de l'activité du patient.

Nous allons maintenant décrire les capteurs 7 pouvant être utilisés seuls ou en combinaison, pour mesurer l'activité du patient de manière à pouvoir comparer ces informations avec le modèle prédictif des fuites urinaires et faire varier la pression de la manchette au niveau de l'urètre en conséquence.

Comme il a déjà été remarqué, il sera préférentiellement utilisé des capteurs ne nécessitant pas d'intervention chirurgicale particulière pour leur mise en place. Notons en outre qu'il est également possible d'utiliser des capteurs déjà implantés chez le patient, ou qui seront implantés plus tard pour d'autres dispositifs. Par exemple, dans le cas où un stimulateur cardiaque (souvent désigné par l'appellation anglo-saxonne « pacemaker ») est ou va être implanté, il sera possible de le faire communiquer avec le sphincter urinaire artificiel présenté pour utiliser en particulier les informations sur le rythme cardiaque du patient. Dans ce cas il conviendra d'utiliser un protocole standard de transfert d'information entre les appareils.

Il peut d'abord être intéressant de mesurer la position et le déplacement du patient.

A cette fin, on peut par exemple utiliser un accéléromètre comprenant un ou plusieurs axes de mesures. Préférentiellement on utilisera un accéléromètre ayant trois axes de mesure de manière pouvoir évaluer les mouvements du patient dans les trois directions de l'espace.

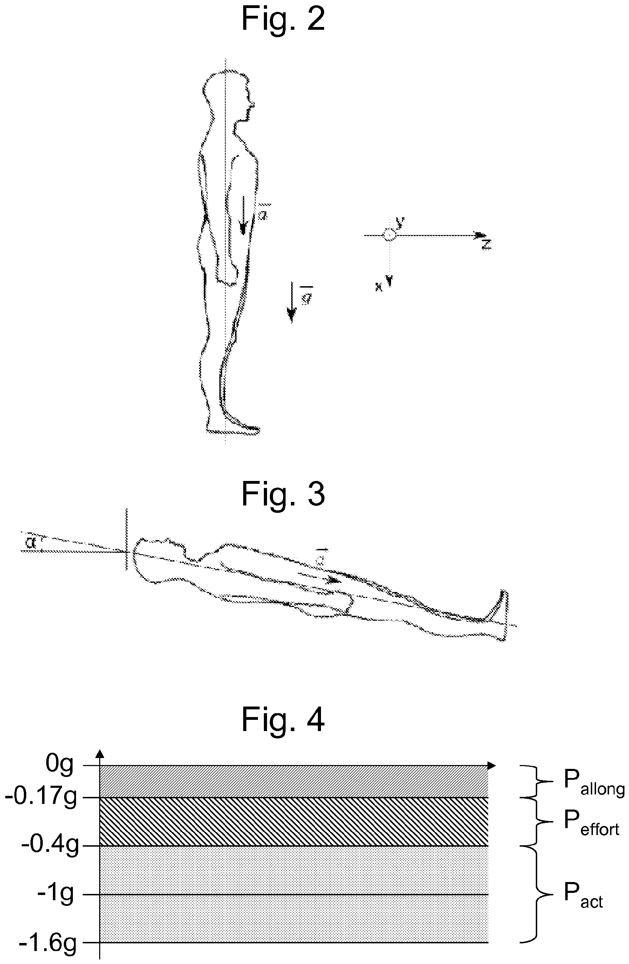

On pourra se contenter d'un accéléromètre ayant un seul axe de mesure, cet accéléromètre étant agencé de manière à ce que l'axe de mesure soit orienté dans l'axe longitudinal du patient, c'est à dire suivant l'axe x tel qu'illustré à la figure 2.

En effet, un accéléromètre à un seul axe de mesure permet de mesurer l'inclinaison du patient grâce à la force de gravité terrestre et ainsi de détecter la position allongée du patient. Il permet aussi d'avoir une information sur les mouvements du patient qui entraîne une augmentation de la pression intra-vésicale, même si cette information n'est pas complète puisqu'une seule direction de mouvement est détectée.

En ce qui concerne la détection de la position allongée, en considérant que la force de gravité g est suivant l'axe x (voir figure 2), une simple mesure de l'accélération $\vec{a}_x$ suivant cet axe permet de savoir si le patient est couché. En effet, en estimant que la position allongée est considérée lorsque $\alpha < 10°$ (voir figure 3), on obtient une amplitude $|\vec{a}_x| = 9.81 \sin(10) = 1.7 m/s^2$ soit environ 0.17g, que l'on prendra comme notre seuil de détection.

La prise en compte du temps pendant lequel l'amplitude de l'accélération est inférieure à 0.17g est aussi significative car cette amplitude peut être aussi inférieure au seuil déterminé sans pour autant que le patient soit allongé. Ce sera par exemple le cas lorsque le patient subit des accélérations relatives suivant les x négatifs (référentiel de la figure 2) pendant un court instant, comme par exemple lorsqu'il est dans un ascenseur ou qu'il fait des sauts. Ainsi, il convient de calculer le temps pendant lequel l'accélération est inférieure au seuil de 0.17g de manière à confirmer un état stable du patient allongé, et d'actionner alors le système pour que la pression dans la manchette passe à la pression $P_{allong}$.

L'accéléromètre est aussi utilisé en tant qu'actimètre pour mesurer les accélérations selon l'axe x qui sont fonction des mouvements du patient. Comme on l'a dit plus haut, des personnes souffrant d'incontinence d'effort (incontinence peu sévère) souffrent souvent de fuites au cours d'activités qui entraînent une augmentation des pressions abdominale, vésicale et urétrale liées aux forces de pressions exercées par les organes environnants. Ces fuites dépendent encore une fois de plusieurs facteurs en relation avec les mouvements du patient. La mesure du mouvement du patient donne une information supplémentaire quant à l'estimation de la pression de la manchette requise, et permet notamment de déterminer la transition de pression $P_{act}$ à $P_{effort}$ qui s'effectue lorsque le patient est en train d'exercer un effort, telle qu'une activité sportive par exemple ou encore lorsque il descend des escaliers.

Des mesures ont été réalisées avec l'accéléromètre pendant diverses activités du patient, afin de déterminer le seuil sur la mesure accélérométrique permettant de différencier l'activité "normale" de l'activité à "l'effort". Le capteur accélérométrique à un axe a été placé sur l'abdomen d'une personne en train de marcher, de courir et de sauter et les données ont été enregistrées au cours de ces activités. Le tableau ci-dessous décrit les accélérations notées lors des divers exercices réalisés, on notera que lorsque le patient est debout sans bouger, avec l'accéléromètre dirigé dans le sens des x (selon le référentiel de la figure 2), la mesure est négative puisque la masselotte mobile utilisée dans l'accéléromètre subit la force de gravité terrestre.

| Posture/activité | Accélération (g) |
|---|---|
| Debout | -1 |
| Allongé | 0 |
| Marche | ±0.5 |
| Course | > \|0.7\| |
| Saut, faible amplitude | > \|0.7\| |
| Saut, grande amplitude | >\|1.5\| |
| Escalier (descente) | ≈\|0.7\| |

Ces différentes mesures ont permis de déterminer plusieurs seuils de déclenchement permettant de relier une accélération mesurée à un type d'activité, et donc à une pression de manchette associée.

Un autre type de capteurs pouvant être utilisé sont des capteurs de pression.

On sait que les fuites sont dépendantes de la pression intra-vésicale. Le moyen le plus simple de construire le modèle prédictif et de modifier ensuite la pression de la manchette serait de mesurer cette pression intra-vésicale. La mise en place de capteurs intra-vésicaux est toutefois difficile ce qui amène à utiliser d'autres types de capteurs, en complément ou en substitution.

L'installation d'un capteur de pression entre l'os du pubis et la paroi vésicale permet de mesurer indirectement la pression dans la vessie. Les mesures de ce capteur de pression enchâssé dans la paroi vésicale permettent une bonne corrélation avec la pression intra-vésicale réelle. Malgré les faibles amplitudes de mesure, il est donc possible d'obtenir les valeurs de pression intra-vésicale à partir de ce capteur particulier.

Il a en outre été mis en évidence qu'un effort de poussée abdominale entraînait une augmentation de la pression vésicale, ce qui, au-delà d'un certain seuil de pression abdominale, pouvait entraîner une fuite urinaire. Il est donc intéressant d'utiliser un capteur de pression abdominal pour pouvoir anticiper une fuite urinaire éventuelle. Il est à noter que la valeur seuil de la pression abdominale correspondant à une fuite urinaire dépend du volume de remplissage de la vessie. Ainsi le calibrage de la valeur seuil de la pression abdominale correspondant à une fuite urinaire pourra être effectué en considérant une valeur moyenne de remplissage de la vessie. Comme on le verra plus loin, on pourra également prévoir un système permettant de mesurer ou estimer le taux de remplissage de la vessie, pour avoir ainsi des valeurs seuils de pression abdominale fonction da la réplétion de la vessie.

La détermination de la ou des valeurs seuils de pression abdominale pour la fuite urinaire peut être faite de la manière suivante. On fixe la pression de la manchette à $P_{act}$ et on demande ensuite au patient d'effectuer un effort de poussée abdominale à glotte fermée de manière progressivement croissante jusqu'à observation d'une fuite (la pression dans la manchette reste à $P_{act}$). A cet instant, la valeur de la pression abdominale est enregistrée et correspondra à la pression abdominale seuil $P_{abdoseuil}$ (dont une marge sera soustraite à cette valeur) qui déclenchera le passage de $P_{act}$ à $P_{effort}$ de la manchette occlusive. Cet exercice est réalisé pour différents volumes de remplissage de la vessie ce qui permettra d'obtenir une valeur de seuil $P_{abdoseuil}$ en rapport avec le volume estimé.

Un capteur de pression urétrale pourra également être prévu. Le but sera essentiellement sécuritaire, pour déterminer une augmentation anormale de pression dans l'urètre, par exemple lors de l'introduction d'une sonde endo-urétrale, de sorte que la manchette soit actionnée pour ne plus contraindre l'urètre de manière à autoriser un passage de la sonde sans contrainte. A cette fin, on peut par exemple utiliser un capteur de pression situé sur la paroi de la manchette en contact avec l'urètre, de manière à pouvoir suivre les variations de pression au niveau de l'urètre. En effet, une augmentation importante de la pression serait le signe qu'un objet est probablement en train d'être introduit au travers de l'urètre.

La mesure de l'activité des muscles permet également de prévoir des fuites urinaires éventuelles. A cette fin, on peut utiliser des capteurs MMG (mécanomyographe) destinés à être placés sur le muscle d'intérêt pour mesurer les mouvements générés lors de contractions dudit muscle. On peut également utiliser des capteurs EMG (électromyographe) destinés à être placés à travers le muscle pour mesurer le potentiel électrique généré lors de contractions dudit muscle.

La continence chez un sujet sain est satisfaite par une synchronisation musculaire, en particulier entre les muscles grand droit de l'abdomen et le plancher périnéal. Il a été démontré que le temps entre une augmentation de la pression intra-vésicale et une augmentation de la pression intra-urétrale est évalué à $t_p \approx 250ms \pm 100ms$ avec une pression urétrale dépassant la pression abdominale. Cela signifie qu'il existe une anticipation de la contraction du sphincter urinaire sur l'augmentation brutale de la pression abdominale pour éviter la fuite. Lors d'une toux, par exemple, le sphincter se contracte au moment où cette toux se fait ressentir.

Si on utilise des capteurs implantés sur au moins une portion de l'un des muscles grand droit, par exemple le muscle grand droit de l'abdomen, et si on estime que le délai mécano-électrique (c'est à dire le délai entre le début d'une activité électrique du muscle et le début d'une activité mécanique) est du même ordre que $t_p$, alors il est possible de prévoir des augmentations de pressions brutales dans la vessie.

Des mesures de l'activité du détrusor, qui est le muscle constituant la vessie, donnent également des informations directement exploitables pour l'anticipation d'une fuite urinaire.

Le plancher pelvien constitue avec les os du bassin le bas de la cavité abdominale, il contient en partie l'urètre et est formé par un groupe complexe de muscles, de ligaments et de nerfs. Lors d'efforts, c'est grâce à cet ensemble d'organes que la continence chez un sujet sain est satisfaite. La réduction des fuites urinaires chez une personne souffrant d'incontinence peu sévère, comme l'incontinence d'effort, est possible après une rééducation du plancher pelvien. Cette rééducation est traduite principalement par un travail musculaire visant à renforcer le tonus du plancher pelvien. Les résultats après une rééducation ont montré une augmentation de la force des muscles périnéaux et une bonne amélioration de l'incontinence chez des femmes souffrant d'incontinence d'effort. Dans la même optique que la rééducation du périnée, nous proposons d'implanter une sonde EMG, pour acquérir les contractions musculaires périnéales dont le temps et l'amplitude seront corrélés à la consigne de pression de la manchette. En effet, une rééducation spécifique du plancher pelvien permet au patient, à partir des contractions musculaires qu'il effectue, de commander le système pour que la pression dans la manchette passe à $P_{effort}$ lorsqu'il ressent une fuite. Il s'agit en fait de fournir un paramètre supplémentaire au système dans le cas particulier où il n'aurait pas réagi à la consigne pour éviter cette fuite. On peut imaginer à titre d'exemple que si le patient ressent une fuite urinaire, il contracte les muscles du périnée ce qui amène la pression dans la manchette à $P_{effort}$ et arrête ainsi la fuite. La contraction des muscles périnéaux est un réflexe naturel lorsque le besoin mictionnel se fait de plus en plus ressentir. Par conséquent, la "commande" de la pression de la manchette en fonction de l'activité du plafond pelvien ne nécessite pas une rééducation compliquée et difficile à assimiler par le patient.

L'EMG du plancher pelvien peut aussi être utilisé pour la commande de l'ouverture de la prothèse (passage de la pression de la manchette à $P_0$) lorsque le patient contracte x fois tout les y secondes les muscles du périnée. La séquence de contractions des muscles périnéaux permettant l'ouverture de la manchette se doit d'être suffisamment complexe pour éviter le risque d'obtenir une même séquence qui serait involontaire mais suffisamment simple pour avoir une reproductibilité de la séquence des contractions.

Comme il a été indiqué plus haut, la connaissance du remplissage de la vessie peut être un paramètre intéressant à prendre en compte, notamment pour augmenter la précision des corrélations entre les mesures de capteurs par rapport à la prédiction d'une fuite.

En effet, après la miction complète, la vessie est quasiment vide, il n'est donc pas nécessaire d'appliquer la même pression sur l'urètre que lorsque la vessie était pleine, que ce soit pour $P_{act}$ ou bien pour $P_{effort}$. C'est pourquoi les deux valeurs de pression $P_{act}$ et $P_{effort}$ augmentent dans le temps après la miction du patient jusqu'à atteindre les seuils déterminés préalablement (pour arriver à Pactmax et $P_{effortmax}$ juste avant la miction suivante).

Une mesure réelle de la réplétion de la vessie peut difficilement être mise en oeuvre. Il est toutefois possible d'estimer le remplissage de la vessie. En effet, la fréquence mictionnelle de la personne (évaluée à environ 4 à 5 fois par 24 h sans miction nocturne chez un sujet sain) est enregistrée, ce qui permet d'estimer le temps moyen de remplissage de la vessie, c'est à dire le temps pour passer d'une pression vésicale quasiment nulle à la pression $P_{actmax}$ pour une activité dite "normale" du patient. On aura donc un temps moyen un temps moyen $T_{moy}$ tel que :

$$T_{moy} = \sum \frac{T_m(i)}{k}$$

avec $T_m(i)$ le i ème temps enregistré entre deux mictions (i allant de 1 à k) et k le nombre de périodes mictionnelles enregistrées.

Au départ, c'est à dire après l'implantation, le temps $T_{moy}$ est de courte durée, il peut être éventuellement estimé en fonction du patient. Ensuite, au fur et à mesure des enregistrements, $T_{moy}$ correspondra de plus en plus au temps moyen entre chaque miction du patient. Il est clair que selon l'activité du patient et son hydratation, le remplissage de la vessie est plus ou moins rapide. Toutefois, la pression exercée sur l'urètre sera toujours suffisante grâce à la prise en compte d'une marge supérieure de pression appliquée et des réglages effectués auparavant par le personnel soignant sur les pressions de clôture nécessaires. En supposant que la fréquence de miction suit une loi gaussienne, on peut par exemple faire croître les valeurs de la consigne de pression linéairement jusqu'à atteindre leur maximum à $T_{moy}$ - a, avec a l'écart type de notre fonction.

Une alternative pour estimer le degré de remplissage de la vessie consiste à utiliser un capteur échographique miniature, par exemple construit sur la technologie CMUT, c'est à dire utilisant un transducteur capacitif à ultrasons micro-usiné. La vessie est en effet caractérisée par une faible échogénéicité. Le capteur est implanté de manière à pouvoir observer la vessie. Dans l'idéal, une observation bidimensionnelle (voire tridimensionnelle) est réalisée, mais il faut noter qu'une observation unidimensionnelle peut apporter une information suffisante. L'avantage d'une observation selon une seule direction est que le traitement des données (qui consiste par exemple à mesurer le nombre de points dont l'échogénéicité est au-dessous d'un certain seuil) est extrêmement simple et nécessite très peu de puissance de calcul. Ce nombre de points peut être corrélé au degré de remplissage de la vessie. La fonction de corrélation peut être estimée au début, et ensuite affinée lors de l'utilisation du dispositif par le patient, grâce à l'analyse par le patient et par l'équipe médicale de l'évolution du résultat du traitement des données échographiques en fonction d'observations cliniques (fuites urinaires, par exemple) ou de mesures du degré de remplissage de la vessie par d'autres moyens (échographie externe réalisée par un médecin lors d'une consultation, par exemple).

On remarquera qu'en prenant en compte la vitesse de remplissage de la vessie, on réduit aussi la consommation énergétique du système étant donné que les actionneurs sont moins sollicités.

La fonction principale des capteurs décrits en détail ci-dessus est de permettre la construction d'un modèle prédictif de fuites urinaires qui sera utilisé pour la gestion des modifications de pression sur l'urètre en fonction de l'activité du patient.

Toutefois, ces capteurs peuvent également être utilisés pour commander le dispositif de prévention de fuites urinaires présenté. En effet, lorsque le patient veut uriner, il convient de commander le dispositif pour que la manchette atteigne une pression $P_0$ permettant une miction sans contrainte. Plutôt que d'utiliser un actionneur mécanique, les capteurs peuvent être utilisés pour transmettre un signal de miction donné par l'utilisateur au dispositif de commande 2.

Cette alternative a déjà été présentée plus haut dans le cas des capteurs d'activité des muscles pelviens. On peut en effet programmer le dispositif de commande 2 pour qu'il actionne le dispositif en vue d'une pression de miction $P_0$ en réponse à une succession particulière de signaux issus du capteur EMG des muscles pelviens.

Ceci est réalisable avec la plupart des autres capteurs.

Il convient en outre qu'une tierce personne puisse actionner le dispositif de prévention de fuites urinaires dans le cas ou le patient n'est plus apte à l'activer seul ou pour toute autre raison.

Comme on l'a dit plus haut, le dispositif de commande 2 est de préférence adapté pour une communication sans fil avec un dispositif de commande externe 6. Ce dispositif de commande externe pourra par exemple être adapté pour imposer au dispositif de commande 2 d'actionner la manchette 1 en vue d'une pression de miction $P_0$.

Tout type de communication peut être envisagé tel qu'une communication radiofréquence.

Une solution alternative ou complémentaire est d'utiliser les capteurs comme une commande d'ouverture du dispositif, de la même manière que cela est prévu pour le patient. Par exemple, en utilisant un capteur de la pression abdominale et en programmant une séquence d'ouverture particulière, une tierce personne pourra commander l'ouverture du sphincter urinaire artificiel en tapant sur l'abdomen selon la séquence particulière.

Le système pourra également être pourvu d'une valve de sécurité actionnable par un champ magnétique, et qui sera adaptée pour mettre la manchette sous une pression autorisant la miction en cas d'actionnement par un système à aimantation externe particulier. L'utilisation d'un système à aimantation présente l'avantage d'autoriser une ouverture sans nécessiter de consommation d'énergie.

Le dispositif de prévention de fuites urinaires présenté ici offre de nombreux avantages pour le traitement de l'incontinence urinaire, quel qu'en soit le degré de gravité.

Les facilités de commande et les sécurités de fonctionnement sont tout d'abord importantes pour le confort de l'utilisateur.

En outre l'automatisation de la prothèse par l'utilisation de moyens de commande électronique réglables à distance par un dispositif externe offre la possibilité d'ajuster les réglages du dispositif sans avoir à opérer, ce qui est particulièrement avantageux quand on sait que les contraintes de pression nécessaires au niveau de l'urètre, ou les valeurs seuils de pression de fuites, peuvent évoluer dans le temps.

Enfin, l'avantage le plus substantiel réside dans le fonctionnement dynamique que permet le dispositif décrit. Le fait de pouvoir exercer une pression sur l'urètre dépendant des besoins réels du patient, en fonction de l'activité exercée, permet une sollicitation moindre de l'urètre, et réduit ainsi les risques d'atrophie urétrale.

A titre illustratif, en considérant un sphincter urinaire artificiel de l'art antérieur exerçant une pression de l'ordre de 70 $cmH_2O$ lorsqu'il est activé, et en admettant que la fréquence de miction du patient est d'environ 4 fois par 24 heures et que chaque miction dure environ 3 minutes au cours desquelles l'ouverture de la manchette est quasi nulle, on obtient une pression moyenne exercée sur l'urètre d'environ 69.8 $cmH_2O$. Si on considère les mêmes paramètres pour la prothèse présentée ici, avec des pressions réglées après l'implantation à $P_{allong}$ = 30 $cmH_2O$ pendant 30% du temps, $P_{act}$ = 45 $cmH_2O$ 50% du temps et $P_{effort}$ = 70 $cmH_2O$ pendant les 20% restant, on obtiendrait une pression moyenne dans la manchette d'approximativement 45.5 $cmH_2O$ soit une réduction de la pression appliquée sur l'urètre de près de 36%.

Le lecteur aura compris que de nombreuses modifications peuvent être apportées sans sortir matériellement des nouveaux enseignements et des avantages décrits ici. Par conséquent, toutes les modifications de ce type sont destinées à être incorporées à l'intérieur de la portée du dispositif de prévention de fuites urinaires comme définie par les revendications adjointes.

**Revendications**

1. Dispositif de prévention de fuites urinaires destiné à être implanté chez un patient, comprenant :

   - des moyens de compression (1,3,4,5) de l'urètre du patient,
   - des moyens de commande électronique (2) pour actionner les moyens de compression (1,3,4,5),

   **caractérisé en ce qu'**il comprend en outre des moyens de mesure (7) de l'activité du patient couplés aux moyens de commande (2), les moyens de commande (2) fonctionnant suivant un modèle prédictif des fuites urinaires basé sur l'activité du patient, de manière à anticiper une fuite urinaire éventuelle en fonction de l'activité mesurée du patient.

2. Dispositif selon la revendication 1, **caractérisé en ce que** les moyens de mesure (7) comprennent des moyens de mesure de la position et du déplacement du patient.

3. Dispositif selon la revendication 2, **caractérisé en ce que** les moyens de mesure de la position et du déplacement du patient comprennent un accéléromètre comprenant un ou plusieurs axes de mesure.

4. Dispositif selon l'une quelconque des revendications 1 à 3, **caractérisé en ce que** les moyens de mesure (7) comprennent des moyens de mesure de la pression intra-abdominale du patient.

5. Dispositif selon l'une quelconque des revendications 1 à 4, **caractérisé en ce que** les moyens de mesure (7) comprennent des moyens de mesure de la pression intra-vésicale.

6. Dispositif selon l'une quelconque des revendications 1 à 5, **caractérisé en ce que** les moyens de mesure (7) comprennent des moyens de mesure de la pression endo-urétrale du patient.

7. Dispositif selon l'une quelconque des revendications 1 à 6, **caractérisé en ce que** les moyens de mesure (7) comprennent des moyens de mesure de l'activité d'un muscle.

8. Dispositif selon la revendication 7, **caractérisé en ce que** les moyens de mesure de l'activité d'un muscle comprennent un capteur MMG (mécanomyographe) destiné à être placé sur ledit muscle pour mesurer les mouvements généré lors de contractions dudit muscle.

9. Dispositif selon l'une quelconque des revendications 7 ou 8, **caractérisé en ce que** les moyens de mesure de l'activité d'un muscle comprennent un capteur EMG (électromyographe) destiné à être placé à travers ledit muscle pour mesurer le potentiel électrique généré lors de contractions dudit muscle.

10. Dispositif l'une quelconque des revendications 7 à 9, **caractérisé en ce que** les moyens de mesure de l'activité d'un muscle comprennent des moyens pour la mesure de l'activité d'au moins une portion d'un des muscles grands droits.

11. Dispositif l'une quelconque des revendications 7 à 10, **caractérisé en ce que** les moyens de mesure de l'activité d'un muscle comprennent des moyens pour la mesure de l'activité d'au moins un des muscles pelviens.

12. Dispositif l'une quelconque des revendications 7 à 11, **caractérisé en ce que** les moyens de mesure de l'activité d'un muscle comprennent des moyens pour la mesure de l'activité du détrusor.

13. Dispositif selon l'une quelconque des revendications 1 à 12, **caractérisé en ce que** les moyens de mesure comprennent des moyens de mesure de la fréquence cardiaque du patient.

14. Dispositif selon l'une quelconque des revendications 1 à 13, **caractérisé en ce que** les moyens de mesure comprennent des moyens de mesure de la fréquence respiratoire du patient.

15. Dispositif selon l'une quelconque des revendications 1 à 14, **caractérisé en ce qu'**il comprend en outre :

   - des moyens de détection destinés à être disposés chez le patient pour une détection de fuites urinaires,
   - des moyens de stockage de signaux de mesure et de détection issus respectivement des moyens de mesure et des moyens de détection,
   - des moyens de traitements des signaux de mesure et de détection stockés pendant un temps déterminé correspondant à une période significative de l'activité du patient, pour construire le modèle prédictif de fuites urinaires du patient, en corrélant une combinaison de tout ou d'une partie des signaux de mesure à la présence ou non d'une fuite urinaire ultérieure, de sorte que le modèle prédictif permette d'anticiper une fuite urinaire éventuelle en fonction de l'activité mesurée du patient.

16. Dispositif selon l'une quelconque des revendications 1 à 15, **caractérisé en ce qu'**il comprend en outre des moyens de mesure de réplétion de la vessie du patient, le modèle prédictif des fuites urinaires étant en outre basé sur la réplétion de la vessie du patient.

17. Dispositif selon la revendication 16, **caractérisé en ce que** les moyens de mesure de réplétion de la vessie comprennent un capteur échographique destiné à être implanté chez le patient pour visualiser la vessie.

18. Dispositif selon l'une quelconque des revendications 1 à 17, **caractérisé en ce que** les moyens de commande (2) comprennent des moyens pour actionner les moyens de compression (1,3,4,5) de façon dynamique en fonction de l'activité mesurée du patient.

19. Dispositif selon l'une quelconque des revendications 1 à 18, **caractérisé en ce que** les moyens de compression (1,3,4,5) comprennent des moyens pour exercer sur l'urètre une compression d'intensité variable, allant d'une absence totale de compression jusqu'à une occlusion totale de l'urètre.

20. Dispositif selon l'une quelconque des revendications 1 à 19, **caractérisé en ce qu'**il comprend en outre des moyens de sécurité adaptés pour actionner les moyens de compression (1,3,4,5) en vue d'une absence totale de compression de l'urètre en réponse à une instruction d'ouverture, et, en réponse à une instruction de fermeture, pour actionner les moyens de compression (1,3,4,5) en vue d'une compression de l'urètre égale à la compression précédant l'instruction d'ouverture du patient.

21. Dispositif selon la revendication 20, **caractérisé en**

**ce que** les moyens de sécurité sont couplés à des capteurs physiologiques destinés à être implantés chez le patient, pour permettre au patient de transmettre une instruction d'ouverture ou de fermeture aux moyens de sécurité.

22. Dispositif selon la revendication 21, **caractérisé en ce que** les capteurs physiologiques sont agencés pour la mesure de contraction d'un muscle de sorte que l'instruction d'ouverture ou de fermeture soit fonction de la fréquence de contraction dudit muscle.

23. Dispositif selon l'une quelconque des revendications 20 à 22, **caractérisé en ce que** les moyens de sécurité sont couplés à des moyens de commande externes (6) adaptés pour permettre à une tierce personne de transmettre une instruction d'ouverture ou de fermeture aux moyens de sécurité.

24. Dispositif selon la revendication 23, **caractérisé en ce que** les moyens de sécurité sont aptes à être activés par les moyens de commandes externes (6) par ondes radio.

25. Dispositif selon la revendication 23, **caractérisé en ce que** les moyens de sécurité sont aptes à être activés par les moyens de commandes externes (6) par ondes magnétiques.

26. Dispositif selon l'une quelconque des revendications 20 à 25, **caractérisé en ce que** les moyens de sécurité son couplés à une horloge interne, l'horloge interne permettant d'enregistrer les intervalles de temps entre les mictions du patient, les moyens de sécurité étant adaptés pour actionner les moyens de compression (1,3,4,5) en vue d'une absence totale de compression de l'urètre en cas de dépassement d'un intervalle de temps maximum entre deux mictions.

**Patentansprüche**

1. Vorrichtung zur Verhinderung von Harnabgängen, die dazu ausgelegt ist, bei einem Patienten implantiert zu werden, umfassend:

 - Mittel zum Komprimieren (1, 3, 4, 5) der Harnröhre des Patienten,
 - Mittel zur elektronischen Steuerung (2), um die Mittel zum Komprimieren (1, 3, 4, 5) zu betätigen,

 **dadurch gekennzeichnet, dass** sie außerdem Mittel zum Messen (7) der Aktivität des Patienten, gekoppelt mit den Mitteln zur Steuerung (2), umfasst, wobei die Mittel zur Steuerung (2) gemäß einem Modell zur Vorhersage von Harnabgängen basierend auf der Aktivität des Patienten funktionieren, um einen eventuellen Harnabgang in Funktion der gemessenen Aktivität des Patienten vorwegzunehmen.

2. Vorrichtung nach Anspruch 1, **dadurch gekennzeichnet, dass** die Mittel zum Messen (7) Mittel zum Messen der Position und der Bewegung des Patienten umfassen.

3. Vorrichtung nach Anspruch 2, **dadurch gekennzeichnet, dass** die Mittel zum Messen der Position und der Bewegung des Patienten einen Beschleunigungsmesser umfassen, der eine oder mehrere Messachsen umfasst.

4. Vorrichtung nach einem der Ansprüche 1 bis 3, **dadurch gekennzeichnet, dass** die Mittel zum Messen (7) Mittel zum Messen des intraabdominalen Drucks des Patienten umfassen.

5. Vorrichtung nach einem der Ansprüche 1 bis 4, **dadurch gekennzeichnet, dass** die Mittel zum Messen (7) Mittel zum Messen des intravesikalen Drucks umfassen.

6. Vorrichtung nach einem der Ansprüche 1 bis 5, **dadurch gekennzeichnet, dass** die Mittel zum Messen (7) Mittel zum Messen des endourethralen Drucks des Patienten umfassen.

7. Vorrichtung nach einem der Ansprüche 1 bis 6, **dadurch gekennzeichnet, dass** die Mittel zum Messen (7) Mittel zum Messen der Aktivität eines Muskels umfassen.

8. Vorrichtung nach Anspruch 7, **dadurch gekennzeichnet, dass** die Mittel zum Messen der Aktivität eines Muskels einen MMG-Sensor (Mechanomyograph) umfassen, der ausgelegt ist, um auf dem Muskel platziert zu werden, um die Bewegungen zu messen, die bei den Kontraktionen des Muskels erzeugt werden.

9. Vorrichtung nach einem der Ansprüche 7 oder 8, **dadurch gekennzeichnet, dass** die Mittel zum Messen der Aktivität eines Muskels einen EMG-Sensor (Elektromyograph) umfassen, der ausgelegt ist, um über den Muskel platziert zu werden, um das elektrische Potenzial zu messen, das bei den Kontraktionen des Muskels erzeugt wird.

10. Vorrichtung nach einem der Ansprüche 7 bis 9, **dadurch gekennzeichnet, dass** die Mittel zum Messen der Aktivität eines Muskels Mittel zu Messen der Aktivität von mindestens einem Abschnitt eines der geraden Bauchmuskeln umfassen.

11. Vorrichtung nach einem der Ansprüche 7 bis 10, **da-**

**durch gekennzeichnet, dass** die Mittel zum Messen der Aktivität eines Muskels Mittel zum Messen der Aktivität von mindestens einem der Beckenmuskel umfassen.

12. Vorrichtung nach einem der Ansprüche 7 bis 11, **dadurch gekennzeichnet, dass** die Mittel zum Messen der Aktivität eines Muskels Mittel zum Messen der Aktivität des *Musculus detrusor vesicae* umfassen.

13. Vorrichtung nach einem der Ansprüche 1 bis 12, **dadurch gekennzeichnet, dass** die Mittel zum Messen Mittel zum Messen der Herzfrequenz des Patienten umfassen.

14. Vorrichtung nach einem der Ansprüche 1 bis 13, **dadurch gekennzeichnet, dass** die Mittel zum Messen Mittel zum Messen der Atemfrequenz des Patienten umfassen.

15. Vorrichtung nach einem der Ansprüche 1 bis 14, **dadurch gekennzeichnet, dass** sie außerdem Folgendes umfasst:

- Mittel zum Nachweisen, die ausgelegt sind, um beim Patienten angeordnet zu sein, um Harnabgänge nachzuweisen,
- Mittel zum Speichern von Mess- und Nachweissignalen, die jeweils aus den Mitteln zum Messen und den Mitteln zum Nachweisen stammen,
- Mittel zur Verarbeiten von gespeicherten Mess- und Nachweissignale während einer festgelegten Zeit, die einem bedeutenden Zeitraum der Aktivität des Patienten entspricht, um das Modell zur Vorhersage von Harnabgängen des Patienten zu konstruieren, indem eine Kombination der Gesamtheit oder eines Teils der Messsignale dem Vorhandenseins oder Nicht-Vorhandensein eines weiteren Harnabgangs korreliert wird, so dass das Modell zur Vorhersage ermöglicht, einen eventuellen Harnabgang in Funktion der gemessenen Aktivität des Patienten vorwegzunehmen.

16. Vorrichtung nach einem der Ansprüche 1 bis 15, **dadurch gekennzeichnet, dass** es außerdem Mittel zum Messen der Sättigung der Blase des Patienten umfasst, wobei das Modell zur Vorhersage der Harnabgänge außerdem auf der Sättigung der Blase des Patienten basiert.

17. Vorrichtung nach Anspruch 16, **dadurch gekennzeichnet, dass** die Mittel zum Messen der Sättigung der Blase einen echographischen Sensor umfassen, der ausgelegt ist, um beim Patienten implantiert zu werden, um die Blase zu visualisieren.

18. Vorrichtung nach einem der Ansprüche 1 bis 17, **dadurch gekennzeichnet, dass** die Mittel zum Steuern (2) Mittel umfassen, um die Mittel zum Komprimieren (1, 3, 4, 5) auf dynamische Weise in Funktion der gemessenen Aktivität des Patienten zu betätigen.

19. Vorrichtung nach einem der Ansprüche 1 bis 18, **dadurch gekennzeichnet, dass** die Mittel zum Komprimieren (1, 3, 4, 5) Mittel umfassen, um auf die Harnröhre eine Kompression mit variabler Intensität auszuüben, die von einer vollständigen Abwesenheit von Kompression bis zu einem vollständigen Verschluss der Harnröhre reicht.

20. Vorrichtung nach einem der Ansprüche 1 bis 19, **dadurch gekennzeichnet, dass** sie außerdem Sicherheitsmittel umfasst, die angepasst sind, um die Mittel zum Komprimieren (1, 3, 4, 5) angesichts einer vollständigen Abwesenheit von Kompression der Harnröhre in Reaktion auf einen Öffnungsbefehl zu betätigen, und, in Reaktion auf einen Verschlussbefehl, um die Mittel zum Komprimieren (1, 3, 4, 5) angesichts einer Kompression der Harnröhre zu betätigen, die gleich der Kompression ist, die dem Öffnungsbefehl des Patienten vorausgeht.

21. Vorrichtung nach Anspruch 20, **dadurch gekennzeichnet, dass** die Sicherheitsmittel an physiologische Sensoren gekoppelt sind, die ausgelegt sind, um beim Patienten implantiert zu werden, um dem Patienten zu ermöglichen, einen Öffnungs- oder Verschlussbefehl an die Sicherheitsmittel zu übertragen.

22. Vorrichtung nach Anspruch 21, **dadurch gekennzeichnet, dass** die physiologischen Sensoren angebracht sind, um die Kontraktion eines Muskels zu messen, so dass der Öffnungs- oder Verschlussbefehl eine Funktion der Frequenz der Kontraktion des Muskels ist.

23. Vorrichtung nach einem der Ansprüche 20 bis 22, **dadurch gekennzeichnet, dass** die Sicherheitsmittel an externe Mittel zum Steuern (6) gekoppelt sind, die angepasst sind, um einer dritten Person zu ermöglichen, einen Öffnungs- oder Verschlussbefehl an die Sicherheitsmittel zu übertragen.

24. Vorrichtung nach Anspruch 23, **dadurch gekennzeichnet, dass** die Sicherheitsmittel geeignet sind, um durch die externen Mittel zum Steuern (6) durch Funkwellen aktiviert zu werden.

25. Vorrichtung nach Anspruch 23, **dadurch gekennzeichnet, dass** die Sicherheitsmittel geeignet sind, um durch die externen Mittel zum Steuern (6) durch magnetische Wellen aktiviert zu werden.

**26.** Vorrichtung nach einem der Ansprüche 20 bis 25, **dadurch gekennzeichnet, dass** die Sicherheitsmittel mit einer internen Uhr gekoppelt sind, wobei die interne Uhr ermöglicht, die Zeitintervalle zwischen den Miktionen des Patienten zu registrieren, wobei die Sicherheitsmittel angepasst sind, um die Mittel zum Komprimieren (1, 3, 4, 5) angesichts einer vollständigen Abwesenheit von Kompression der Harnröhre im Fall des Überschreitens eines maximalen Zeitintervalls zwischen zwei Miktionen zu betätigen.

**Claims**

**1.** Device to prevent urinary leakage intended to be implanted in a patient, comprising:

> - compression means (1, 3, 4, 5) to compress the urethra of the patient,
> - electronic control means (2) to actuate the compression means (1, 3, 4, 5),

**characterized in that** it further comprises measurement means (7) to measure the activity of the patient coupled with the control means (2), the control means (2) operating in accordance with a predictive model of urinary leakage based on the activity of the patient, so as to anticipate any urinary leakage depending on the measured activity of the patient.

**2.** Device according to claim 1, **characterized in that** the measurement means (7) comprise means of measuring the position and movement of the patient.

**3.** Device according to claim 2, **characterized in that** the means of measuring the position and movement of the patient comprise an accelerometer comprising one or more measurement axes.

**4.** Device according to any one of claims 1 to 3, **characterized in that** the measurement means (7) comprise means of measuring the intra-abdominal pressure of the patient.

**5.** Device according to any one of claims 1 to 4, **characterized in that** the measurement means (7) comprise means of measuring the intra-vesical pressure.

**6.** Device according to any one of claims 1 to 5, **characterized in that** the measurement means (7) comprise means of measuring the endo-urethral pressure of the patient.

**7.** Device according to any one of claims 1 to 6, **characterized in that** the measurement means (7) comprise means of measuring the activity of a muscle.

**8.** Device according to claim 7, **characterized in that** the means of measuring the activity of a muscle comprise an MMG (mechanomyograph) sensor intended to be placed on said muscle to measure the movements generated by contractions of said muscle.

**9.** Device according to either of claims 7 or 8, **characterized in that** the means of measuring the activity of a muscle comprise an EMG (electromyography) sensor intended to be placed through said muscle to measure the electric potential generated by contractions of said muscle.

**10.** Device according to any one of claims 7 to 9, **characterized in that** the means of measuring the activity of a muscle comprise means of measuring the activity of at least one portion of one of the rectus abdominis muscles.

**11.** Device according to any one of claims 7 to 10, **characterized in that** the means of measuring the activity of a muscle comprise means of measuring the activity of at least one of the pelvic muscles.

**12.** Device according to any one of claims 7 to 11, **characterized in that** the means of measuring the activity of a muscle comprise means of measuring the activity of the detrusor.

**13.** Device according to any one of claims 1 to 12, **characterized in that** the measurement means comprise means of measuring the heart rate of the patient.

**14.** Device according to any one of claims 1 to 13, **characterized in that** the measurement means comprise means of measuring the respiratory rate of the patient.

**15.** Device according to any one of claims 1 to 14, **characterized in that** it further comprises:

> - detection means intended to be arranged on the patient to detect urinary leakage,
> - storage means to store measurement and detection signals derived from the measurement means and the detection means respectively,
> - processing means to process measurement and detection signals stored for a determined time corresponding to a significant period of activity of the patient, in order to construct the predictive model of urinary leakage of the patient by correlating a combination of all or part of the measurement signals with the presence or absence of a subsequent urinary leakage, so that the predictive model allows anticipation of a possible urinary leakage depending on the measured activity of the patient.

**16.** Device according to any one of claims 1 to 15, **char-**

**acterized in that** it further comprises means of measuring filling of the bladder of the patient, the predictive model of urinary leakage being further based on filling of the bladder of the patient.

17. Device according to claim 16, **characterized in that** the means of measuring filling of the bladder comprise an ultrasound sensor intended to be implanted in the patient to visualize the bladder.

18. Device according to any one of claims 1 to 17, **characterized in that** the control means (2) comprise means of actuating the compression means (1, 3, 4, 5) dynamically depending on the measured activity of the patient.

19. Device according to any one of claims 1 to 18, **characterized in that** the compression means (1, 3, 4, 5) comprise means of applying compression of variable intensity to the urethra, ranging from absolutely no compression to full occlusion of the urethra.

20. Device according to any one of claims 1 to 19, **characterized in that** it further comprises safety means adapted to actuate the compression means (1, 3, 4, 5) to obtain full absence of compression on the urethra in response to an opening instruction, and in response to a closing instruction to actuate the compression means (1, 3, 4, 5) to obtain compression of the urethra equal to the compression prior to the opening instruction given by the patient.

21. Device according to claim 20, **characterized in that** the safety means are coupled with physiological sensors intended to be implanted in the patient, so as to enable the patient to transmit an opening or closing instruction to the safety means.

22. Device according to claim 21, **characterized in that** the physiological sensors are arranged to measure the contraction of a muscle so that the opening or closing instruction depends on the frequency of contraction of said muscle.

23. Device according to any one of claims 20 to 22, **characterized in that** the safety means are coupled with external control means (6) adapted to allow an assistant to transmit an opening or closing instruction to the safety means.

24. Device according to claim 23, **characterized in that** the safety means can be actuated by the external control means (6) via radio waves.

25. Device according to claim 23, **characterized in that** the safety means are adapted to be actuated by the external control means (6) via magnetic waves.

26. Device according to any one of claims 20 to 25, **characterized in that** the safety means are coupled with an internal clock, the internal clock allowing the recording of the time intervals between patient urinations, the safety means being adapted to actuate the compression means (1, 3, 4, 5) to obtain total absence of compression on the urethra in the event that a maximum time interval between two urinations is exceeded.

# Fig. 1

# Fig. 2

# Fig. 3

# Fig. 4

**EP 2 185 102 B1**

## RÉFÉRENCES CITÉES DANS LA DESCRIPTION

**Documents brevets cités dans la description**

- WO 0150833 A